# EUROPEAN PATENT SPECIFICATION

(11) **EP 3 256 040 B1**
(45) Date of publication and mention of the grant of the patent: **23.09.2026**
(21) Application number: 16749603.3
(22) Date of filing: 02.02.2016
(51) Int. Cl.: A61B 5/00, A61B 5/145, A61B 5/1468, A61B 5/1486, A61B 5/06, A61B 5/1473, G01N 24/08

(54) **DEVICES AND SYSTEMS FOR DISTINGUISHING CANCEROUS AND NON-CANCEROUS SKIN TISSUE**
VORRICHTUNGEN UND SYSTEME ZUR UNTERSCHEIDUNG VON KANZERÖSEM UND NICHTKANZERÖSEM HAUTGEWEBE
DISPOSITIFS ET SYSTÈMES PERMETTANT DE DIFFÉRENCIER DES TISSUS CANCÉREUX DE TISSUS NON CANCÉREUX

(30) Priority: 11.02.2015 US 201562115027 P
(43) Date of publication of application: 20.12.2017
(73) Proprietor: Abbott Diabetes Care Inc., Alameda, California 94502 (US)
(72) Inventor: HELLER, Adam, Austin, Texas 78701 (US)
(74) Representative: Williams Powell
(86) International application number: PCT/US2016/016175
(87) International publication number: WO 2016/130364

(56) References cited:
- WO-A1-2014/099290
- WO-A1-2014/118258
- WO-A1-2014/133500
- US-A1- 2004 152 997
- US-A1- 2007 026 440
- US-B1- 6 936 424
- US-B2- 7 305 244
- DENIS LAFRANCE: "Near infrared determination of lactate in biological fluids and tissues", 1 January 2003 (2003-01-01), XP055485940, ISBN: 978-0-612-98401-1, Retrieved from the Internet <URL:http://digitool.library.mcgill.ca/webclient/StreamGate?folder_id=0&dvs=1529479985877~445> [retrieved on 20180618]
- PHILIP R. MILLER ET AL: "Multiplexed microneedle-based biosensor array for characterization of metabolic acidosis", TALANTA, vol. 88, 1 January 2012 (2012-01-01), NL, pages 739 - 742, XP055485824, ISSN: 0039-9140, DOI: 10.1016/j.talanta.2011.11.046

## Description

### BACKGROUND

Distinguishing cancerous tissue from a non-cancerous tissue, especially with fine resolution, has profound and varied applications. Determining location of cancerous tissue and/or the shape and/or size with fine precision and accuracy could provide a surgeon with an exact tissue excision map so that the current surgical practice of wide resection could be minimized or eliminated, radiation, chemotherapy or other treatment delivery locations could be refined, etc. It would be especially valuable to precisely determine the coordinates of a cancerous tissue/non-cancerous tissue interface. Knowing the exact location of this interface or margin as it is often called, helps guide a surgeon and ensure cancerous tissue is resected with little or no non-cancerous tissue-or at least minimize the non-cancerous tissue that is intentionally removed to ensure complete removal of the cancerous, for example.

A number of current methods utilize lactate and/or pyruvate as cancer markers. The primary tool for monitoring malignancy through lactate and pyruvate is currently the use of hyperpolarized 13C (carbon 13) magnetic resonance imaging of the lactate and/or pyruvate formed in the body from intravenously injected hyperpolarized 13-C glucose. The method is pretty complex and requires expensive instrumentation.

Therefore, it would be beneficial to have improved methods, devices and systems that determine location of cancerous tissue and/or the shape and/or size and/or coordinates of a cancerous tissue/non-cancerous tissue interface.

WO2014/133500 discloses a system for monitoring a boundary between cancerous and non-cancerous tissue, the system comprising a processing and control unit and a positioning element for determining locations of two or more sensing elements positioned in vivo in a tissue of a subject.

### SUMMARY

Provided herein are in vivo methods, devices and systems (e.g., electrochemical in vivo methods) that utilize lactate and/or pyruvate levels in identifying cancerous tissue in a tissue or organ, in real time. The in vivo methods, devices and systems disclosed herein measure lactate and/or pyruvate signal proportional to the level of lactate and/or pyruvate, or the lactate to pyruvate ratio, at a location in tissue, which allows a determination of the nature and/or extent of cancerous tissue, e.g., whether the location includes cancerous or non-cancerous tissue. In addition, the in vivo methods, devices and systems utilize the lactate and/or pyruvate signal to demarcate the boundary or margin of cancerous tissue. Also disclosed are in vivo methods, devices and systems that demarcate the boundary of cancerous tissue for at least one of biopsy of cancer cells, resection of cancerous and/or non-cancerous tissue, debulking cancerous tissue (i.e., cytoreduction of cancerous tissue), localized treatment (chemotherapy, radiation, and the like) of cancerous and/or non-cancerous tissue, and the like. In certain embodiments, the in vivo methods, devices and systems that measure lactate and/or pyruvate to evaluate tissue are real time electrochemical methods, devices and systems.

In accordance with the present invention there is provided a system for monitoring a boundary between cancerous and non-cancerous tissue as claimed in claim 1. In certain embodiments, systems disclosed herein obtain a difference signal or multiple difference signals from two or more sensing elements (e.g., electrochemical sensing elements) at two or more in vivo locations, wherein each of the two or more sensing elements senses lactate or each of the two or more sensing elements senses pyruvate, determine, using the positioning element, the locations of at least two of the sensing elements, determine the direction and/or magnitude of the decrease or increase in lactate and/or pyruvate signal from the sensing elements, and correlate the difference or differences and the direction or directions (and/or magnitude or magnitudes) with a boundary (also referred to as margin or interface) between cancerous and non-cancerous tissue at the in vivo locations. For example, certain embodiments include in vivo, e.g., electrochemical, methods that detect a differential lactate and/or pyruvate signal between at least two different locations in a tissue, identify one of the locations as having a higher lactate and/or pyruvate signal and at least one of the other locations as having a lower lactate and/or pyruvate signal, and then identify the locations as having cancerous and non-cancerous tissue, respectively. The in vivo methods, devices and systems disclosed herein may be used to determine a boundary of the cancerous tissue as being located between the locations of the sensing elements that detected the lactate and/or pyruvate signals at the at least two different locations..

Other systems, devices, methods, features and advantages of the subject matter described herein will be or will become apparent to one with skill in the art upon examination of the following figures and detailed description. It is intended that all such additional systems, devices, methods, features and advantages be included within this description, be within the scope of the subject matter described herein, and be protected by the accompanying claims. In no way should the features of the example embodiments be construed as limiting the appended claims, absent express recitation of those features in the claims.

### BRIEF DESCRIPTION OF THE DRAWINGS

The details of the subject matter set forth herein, both as to its structure and operation, may be apparent by study of the accompanying figures, in which like reference numerals refer to like parts. The components in the figures are not necessarily to scale, emphasis instead being placed upon illustrating the principles of the subject matter. Moreover, all illustrations are intended to convey concepts, where relative sizes, shapes and other detailed attributes may be illustrated schematically rather than literally or precisely.
Figs. 1A-1C depict a schematic of an example embodiment of an in vivo method for assessing cancerous tissue in a subject.
Figs. 2A-2K depict schematics of an example embodiment of in vivo lactate and/or pyruvate sensing devices insertable into the skin, tissue or organ for sensing lactate and/or pyruvate signals of the tissue in which the devices are inserted.
Fig. 3A is a side view depicting a melanoma in a subject.
Fig. 3B is a side view depicting an example embodiment of an in vivo lactate and/or pyruvate sensing device for sensing lactate and/or pyruvate signals, inserted into the skin at a location that includes the melanoma.
Fig. 3C is a top view depicting an example embodiment of the device as visible on the surface of the skin.
Fig. 4A is a top view depicting an example embodiment of an in vivo lactate and/or pyruvate sensing device that includes a plurality of lactate and/or pyruvate sensing elements arranged in a grid-like pattern.
Fig. 4B is a side view depicting an example embodiment of the lactate and/or pyruvate sensing device of Fig. 4A as inserted in a melanoma and adjacent tissue.
Fig. 5 depicts an exemplary scalpel of the present disclosure.
Fig. 6 depicts an exemplary system of the present disclosure.

### DETAILED DESCRIPTION

Before the present subject matter is further described, it is to be understood that this subject matter is not limited to particular embodiments described, as such may, of course, vary. It is also to be understood that the terminology used herein is for the purpose of describing particular embodiments only, and is not intended to be limiting, since the scope of the present subject matter will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed. The upper and lower limits of these smaller ranges may independently be included in the smaller ranges, and are also encompassed, subject to any specifically excluded limit in the stated range. Where the stated range includes one or both of the limits, ranges excluding either or both of those included limits are also included.

Unless defined otherwise, all technical and scientific terms used herein have the same meaning as commonly understood by one of ordinary skill in the art. Although any methods and materials similar or equivalent to those described herein can also be used in the practice or testing of the present subject matter, certain example methods and materials are now described.

It must be noted that as used herein and in the appended claims, the singular forms "a," "an," and "the" include plural referents unless the context clearly dictates otherwise. Thus, for example, reference to "a lactate and/or pyruvate sensing element" includes a plurality of such lactate and/or pyruvate sensing elements and reference to "the boundary" includes reference to one or more boundaries and equivalents thereof known to those skilled in the art, and so forth. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

The publications discussed herein are provided solely for their disclosure prior to the filing date of the present application. Nothing herein is to be construed as an admission that the present subject matter is not entitled to antedate such publication by virtue of prior invention. Further, the dates of publication provided may be different from the actual publication dates which may need to be independently confirmed.

Described herein are in vivo methods, devices and systems that utilize the presence and/or concentration of lactate and/or pyruvate in tissue to identify cancerous tissue in a tissue or organ, and further demarcate a boundary between cancerous tissue and neighboring non-cancerous or normal tissue, based on the identification.

The in vivo methods, devices and systems disclosed herein detect and/or measure, e.g., electrochemically, lactate and/or pyruvate signals proportional to lactic and/or pyruvic acid levels at a location in tissue in a subject, and determine whether the location includes cancerous tissue or non-cancerous tissue. In addition, the in vivo methods, devices and systems utilize the detected and/or measured lactate and/or pyruvate signals to demarcate one or more boundaries between cancerous and non-cancerous tissue. Demarcating one or more boundaries between cancerous and non-cancerous tissue facilitates at least one of biopsy of cancer cells, resection of cancerous and/or non-cancerous tissue, debulking cancerous tissue (i.e., cytoreduction of cancerous tissue), localized treatment of cancerous and/or non-cancerous tissue, and the like in a subject.

The methods, devices and systems described herein are used to detect lactate and/or pyruvate signals at multiple locations in tissue or an organ known or at least suspected to have cancerous tissue, use the detected lactate and/or pyruvate signals to identify location of cancerous tissue and non-cancerous tissue, and provide a perimeter within which the identified cancer tissue is located. The method, device and system embodiments described herein can distinguish between cancerous and non-cancerous tissue without the use of an isotope, such as the hyperpolarized 13C (carbon 13) that is used in current marking techniques. Accordingly, each and every step, programming instruction, or functional capability set forth herein can be described as being accomplished without the use of an isotope.

In certain embodiments, one or more lactate and/or pyruvate sensing elements is positioned at one or more locations in and/or around tissue known or suspected of being cancerous, such as a malignant or benign tumor, located in tissue in a subject, and lactate and/or pyruvate signals from (or absence thereof) the different locations are analyzed by a lactate and/or pyruvate processing algorithm to determine whether the lactate and/or pyruvate signals at the different locations are different and the direction in which the signals are increasing or decreasing and/or the magnitude of the difference. An algorithm may include an analysis rule that a tumor may have a higher level of lactate and/or pyruvate which may not significantly vary within the tumor, and non-cancerous tissue may have a lower level of lactate and/or pyruvate which may not significantly vary within such tissue. As such, when a first location is determined to include cancerous tissue and a second location is determined to include non-cancerous tissue, a differential lactate and/or pyruvate signal between the two locations is determined. The direction of decrease points to the region in the tissue that is the boundary or at least closer to the boundary between the cancerous and non-cancerous tissue. Conversely, when the first location includes non-cancerous (e.g., normal tissue) and the second location includes cancerous tissue, determination of a differential signal and a direction in which the signal is increasing points to the region at which a boundary exists or to a region that at least is closer to the boundary between the non-cancerous and cancerous portions of the tissue. Thus, the direction of decrease or increase in the lactate and/or pyruvate signals may be used to establish the edges of cancerous tissue in a subject. Differential signal magnitude can also be included in the process of identifying edges of cancerous tissue in a subject. In certain embodiments, the lactate and/or pyruvate signal processing algorithm includes nearest neighbor processing, spline interpolation, and the like. For example, e.g., a principal component processing, a *K* nearest neighbor (NN) processing, a weighted distance nearest neighbor processing, etc.

There have been several EEG classification studies within the recent years. These studies used different classification techniques, compared their performance, and evaluated different combinations of feature sets. Among these classifiers, k-nearest neighbor (k-NN), linear discriminant analysis (LDA), support vector machine (SVM), artificial neural network (ANN) have been popular. Boostani et al. (Expert Systems with Applications, 36 (2008), pp. 6492-6499) used five different classification algorithms including LDA, Boosted version of direct LDA (BDLDA), Adaboost, SVM, and fuzzy SVM to classify two schizophrenic and normal groups. Their result showed the BDLDA method achieved slightly better performance than the other classification methods. Hazarika et al. (Signal Processing, 59 (1997), pp. 61-72) applied the three-layered ANN using wavelet transform as a feature extraction method for classifying of three groups: normal, schizophrenia, and obsessive compulsive disorder. Their results showed the wavelet transform can be used as a powerful technique for preprocessing EEG signals prior to classification. Li and Fan, (Proceedings of the 2005 IEEE Engineering in Medicine and Biology, 27th Annual Conference Shanghai, September 1-4, (China) 2005) studied the classification of three kinds of subjects (10 schizophrenic patients, 10 depressive patients and 10 normal controls) with EEG rhythms used as feature vectors. They used two ANN approaches, BP ANN and self-organizing competitive ANN for classification. Their results showed that BP ANN has a better comprehensive performance than the self-organizing competitive ANN technique.

Homero et al. (IEEE Transaction on Biomedical Engineering, 53 (2006), pp. 210-218) used three nonlinear methods of time series analysis for analyzing the time series generated by 20 schizophrenic patients and 20 control subjects. Their results show that the ability of generating random time series between schizophrenic subjects and controls is different. The patient group is characterized by less complex neurobehavioral and neuropsychologic measurements. Rosenberg et al. (Psychological Medicine, 20 (1990), pp. 953-960) studied a random number generation experiment. They asked the participant to choose a random number in interval [1..10] without any generative rule. They found that schizophrenic patients tended to be more repetitive. AlZoubi et al. (AlZoubi, O., Calvo, R.A., Stevens, R.H., 2009. In: Nicholson, A., Li, X. (Eds.), Classification of EEG for affect recognition an adaptive approach, Lecture Notes in Computer Science 5866, pp. 52-61) evaluated three different classifier techniques to classify the EEG signals in a 10-class emotion experiment. Their results showed using the adaptive algorithm can improve the performance of the classification task.

The main problem in the classification of EEG signals is the quality of the recorded signal, which can be different during the experiment. These unwanted disturbances cannot be controlled since many activities are going on at the same time in the brain. Existence of artifacts at the time of recording the EEG signal, directly affects the reliability of the recorded signal. Using adaptive classifiers can be useful for the biological signals such as EEG. In this paper, a general adaptive method named weighted adaptive nearest neighbor (WDNN) (Zolghadri et al., Information Sciences, 179 (2009), pp. 2964-2973) is applied for EEG signal classification. This classifier assigns a weight to each training sample that controls its influence in classifying test samples.

In certain embodiments, the lactate and/or pyruvate signals (e.g., electrochemical) obtained from one or more in vivo positioned lactate and/or pyruvate sensing elements may be detected at one or more locations spaced apart from a reference location or from a plurality of reference locations. For example, the at least one reference location may be located in a cancerous tissue, or at least tissue suspected of being cancerous. The one or more locations at which lactate and/or pyruvate signals may be detected may be in the same linear dimension as the reference location, e. g., in a first direction spaced apart from the reference location and in the same plane. In certain cases, the plurality of locations at which lactate and/or pyruvate signals may be detected may be in one or more different dimension from the reference location, e. g., in a plane perpendicular (or at any angle) to the plane at which the reference location is positioned. In certain embodiments, the lactate and/or pyruvate signals may be detected at locations spaced apart from a reference location or from a plurality of reference locations, where the different locations may be in a first, a second, and a third direction using the reference location as the origin where virtual lines from the first, second, and third directions intersect. Detection of lactate and/or pyruvate signals in three dimensions about a reference location facilitates mapping the shape of the cancerous tissue and demarcating the edges of the cancerous tissue. For example, determining the boundary of a cancerous tissue may include analyzing lactate and/or pyruvate signal from one, two, three or higher dimensional space locations in and around the cancerous tissue, and generating a map such as a three dimensional map of the cancerous tissue and/or the surrounding non-cancerous tissue.

In certain aspects, the methods, devices and systems herein may include inserting one or more lactate and/or pyruvate sensing elements at a plurality of locations in a tissue, detecting lactate and/or pyruvate signals at the plurality of locations, determining the direction and/or magnitude in which the detected signals are decreasing (e.g., in one or more of X-Y-Z dimensions), correlating the signals to the locations by obtaining information regarding the position of the different locations in the tissue, mapping the locations in the tissue, and ascertaining the edges of the cancerous tissue. The methods, devices and systems herein also include a visual representation of the processed signal information on a display device. For example, the lactate and/or pyruvate data can be used to generate a topographical display of the sensed tissue and the features thereof.

Embodiments include in vivo methods, devices and systems for assessing one or more edges of a tumor adjacent to non-cancerous cells in the tissue in which the tumor is present, e.g., electrochemically. The in vivo methods, devices and systems may be used for, for example, detecting a first lactate and/or pyruvate signal at a first location in tissue in a subject, detecting a second lactate and/or pyruvate signal at a second location in the tissue, determining whether the first signal differs from the second signal and if so, if the first signal is higher than the second signal, determining the first location as including cancerous tissue and the second location as including non-cancerous tissue, and determining a boundary between the cancerous and non-cancerous tissues that is at a location between the first and second locations. This process can be repeated multiple times at the same or different first and/or second locations. The designation of a first location and second location does not imply a particular sequence and is used to imply relative positions and may be used reversibly.

In certain cases, determining cancerous/non-cancerous tissue based on the obtained differential lactate and/or pyruvate signal may include comparing the difference between the first and second signals to a reference value. The reference value may be the difference between a lactate and/or pyruvate signal in a tissue known to be cancerous and a lactate and/or pyruvate signal from a tissue known to be non-cancerous. In certain embodiments, the reference value may be based on lactate and/or pyruvate signals obtained from the same tissue type and the same cancer type as the tissue and cancer being assessed in the in vivo method.

In certain cases, determining cancerous/non-cancerous tissue based on the obtained differential lactate and/or pyruvate signal may include comparing the first signal and the second signal to a threshold signal indicative of non-cancerous tissue, wherein the first signal is higher than the threshold signal and the second signal is lower than the threshold signal. The threshold signal may be a signal known or empirically determined to be indicative of non-cancerous tissue. For example, the threshold signal may be a signal that distinguishes non-cancerous tissue from cancerous tissue, where the threshold signal is for the same type of tissue and/or cancer as being assessed in the methods disclosed herein. In certain cases, the threshold signal may be determined by measuring lactate and/or pyruvate signals from a plurality of locations in the tissue in a subject which locations are known not to include cancerous tissue. For example, the method may include detecting lactate and/or pyruvate level-related signals from a plurality of locations in tissue in a subject which tissue is being assessed for demarcating a boundary of a tumor present in the tissue. The plurality of locations may include locations known to be non-cancerous, for example, tissue that is significantly spaced apart from the tumor and hence not likely to be cancerous. The plurality of locations may include locations known to be cancerous, for example, tissue that is present in a location known to be in or on the tumor. These measurements provide a threshold or reference level of lactate and/or pyruvate signal that clearly distinguishes cancerous from non-cancerous tissue. These measurements also provide a lactate and/or pyruvate level associated with a negative control (i.e., non-cancerous tissue) and a lactate and/or pyruvate level associated with a positive control (i.e., cancerous tissue).

In certain embodiments, the methods include detecting lactate and/or pyruvate signals along a first direction and a second direction in the tissue, and generating a two-dimensional map of the tumor based on the detection. In certain embodiments, the lactate and/or pyruvate signals may be detected along a first direction, a second direction, and a third direction to determine a three-dimensional map of the tumor based on the detected signals. In certain embodiments, the first and second directions may be perpendicular to each other at the point at which virtual lines drawn in the first and second directions intersect, or the second direction may be at any angle relative to the first direction. In certain embodiments, the first, second, and third directions may be perpendicular to each other at the point at which virtual lines drawn in the first, second, and third directions intersect, or at any angle relative to one another.

In certain embodiments, lactate and/or pyruvate signals may be detected at a plurality of in vivo locations in tissue using a lactate and/or pyruvate sensor or detector device that includes one or more lactate and/or pyruvate sensing elements. In certain embodiments, lactate and/or pyruvate signals may be detected at a plurality of in vivo locations in a tissue using a lactate and/or pyruvate detector device that includes a single or multiple lactate and/or pyruvate sensing elements which is sequentially moved to the plurality of locations. In certain embodiments, lactate and/or pyruvate signals may be detected at a plurality of locations sequentially or simultaneously or a combination thereof. Movement of the sensing element(s) may be manual or automatic, e.g., under the control of a programmed lactate and/or pyruvate sensing controller programmed to carry out one or more of the processes disclosed herein. The lactate and/or pyruvate sensing controller may determine the sensing locations, e.g., based on information obtained by the sensing device using the process, i.e., it may iteratively process lactate and/or pyruvate sensing data obtained and determine one or more subsequent lactate and/or pyruvate sensing locations based on the data, and may automatically move one or more sensing elements to the determined location(s), and repeat the lactate and/or pyruvate sensing process one or more times, e.g., until the device determines that the complete cancerous margin has been mapped.

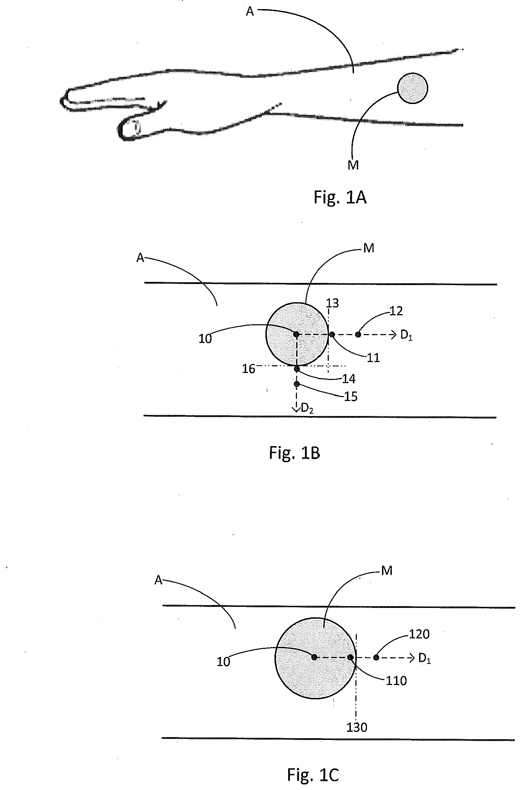

In certain embodiments, the in vivo methods, devices and systems of assessing lactate and/or pyruvate tissue may include determining a location in tissue at which cancerous tissue is located or at least suspected of being located, and positioning one or more lactate and/or pyruvate sensing elements thereat in the subject, and initiating in vivo lactate and/or pyruvate sensing. This location may be designated as a reference location. The lactate and/or pyruvate sensing element or another lactate and/or pyruvate sensing element may be inserted at a location spaced apart from the reference location and in the same or different plane as the reference location and detecting the lactate and/or pyruvate signal at this location. This location may be designated as the first location. A decreased lactate and/or pyruvate signal at this first location compared to the reference location is determined to indicate that this first location represents an edge of the cancer tissue. Also included may be detecting lactate and/or pyruvate signal at a second location which is in the same or different plane as the first location with respect to the reference location. The obtained signal may then be analyzed using a program that applies a rule that decreased lactate and/or pyruvate signal at the second location compared to lactate and/or pyruvate signal at the reference location and lack of difference in the signals detected at the first and second locations confirms that the first and second locations correspond to non-cancerous tissue. Thus, the region of the tissue in-between the reference location and the first location is determined to be a boundary between cancerous and non-cancerous tissue. A similar procedure may be performed in another direction with respect to the reference location to map another edge of the cancerous tissue. An embodiment of such a method is described with regard to Figures 1A, 1B and 1C.

In Figure 1A, a melanoma M located on an arm A of a subject is depicted. It is understood that melanoma M is a three dimensional tissue mass in most instances, but is shown in two dimensions here for simplicity. The lactate and/or pyruvate presence and/or concentration analysis disclosed herein can be repeated one or more times in one or more dimensions to determine the complete spatial size/shape of melanoma M in one or more dimensions, e.g., the topography of Melanoma M. Figure 1B is a schematic of the melanoma M in which a lactate and/or pyruvate sensing element (not shown) is inserted at a reference location 10 to obtain a reference lactate and/or pyruvate signal at the reference point 10, which is at or near the center point of melanoma M, but could be positioned elsewhere. A first location 11 is selected spaced apart from the reference location 10 in a first direction D1, a lactate and/or pyruvate sensing element is inserted at the first location 11, and a first lactate and/or pyruvate signal is obtained at the first location 11 from the inserted sensing element. The reference signal and first lactate and/or pyruvate signal may be obtained simultaneously or sequentially. A single lactate and/or pyruvate sensing element may be used (and moved about), or multiple sensing elements may be used. The multiple sensing elements may be separate or joined together as a single sensing unit. The first lactate and/or pyruvate signal from the first location 11 is compared to the reference lactate and/or pyruvate signal at the reference location 10, and the tissue at the reference and first locations 10 and 11 are determined to be cancerous or non-cancerous based on the comparison of such signals. For example, if the first lactate and/or pyruvate signal detected at the first location 11 is determined to be lower than the reference signal at the reference location 10, then it is identified as non-cancerous tissue. This process can be repeated one or more times. A second location 12 spaced-apart from the reference location 10 and the first location 11 may then be selected. This second location 12 is in the direction from the reference and first location 10 and 11 in which the lactate and/or pyruvate signal is decreasing as determined by a lactate and/or pyruvate signal analysis. A determination of whether the lactate and/or pyruvate signal (designated as a second lactate and/or pyruvate signal) detected at this second location 12 correlates to cancerous or non-cancerous tissue is carried-out by comparing it to the reference signal and/or the first lactate and/or pyruvate signal. For example, if the second lactate and/or pyruvate signal is determined to be lower than the reference lactate and/or pyruvate signal and the same as the first lactate and/or pyruvate signal, then the first and second locations are determined to be non-cancerous tissue and an edge/boundary 13 of the cancerous tissue or tumor is confirmed to be at a location between the reference location 10 and first location 11. Iteratively repeating this process to further complete the mapping of the spatial size of the melanoma M, a third location 14 and a fourth location 15, etc., may be similarly assessed. For example in the embodiment of Figure 1B, third and fourth locations 14 and 15 are locations in the same plane as the locations 10, 11, and 12 and are in a second direction D2 perpendicular (or other determined angle) to the first direction D1 in which the first and second locations 11 and 12 were positioned, and a determination of lactate and/or pyruvate concentrations at these locations is performed. For example, if lactate and/or pyruvate signal at the third location 14 is determined to be lower than the reference lactate and/or pyruvate signal at the reference location 10, then it is determined that the second direction D2 is towards the edge of the melanoma M. A lactate and/or pyruvate signal at the fourth location 15 determined to be the same as the lactate and/or pyruvate signal at the third location 14 indicates that the third and fourth locations 14 and 15 are non-cancerous tissue. Thus, a boundary 16 of the melanoma M is located between the reference and third locations 10 and 14. As noted above, this procedure is repeated a number of times sufficient to map a perimeter of the cancerous tissue of the melanoma M. Distances between sensing locations may be uniform or may vary. For example, the distances may be progressively shorter as the locations move farther away from a center point or other reference point of targeted tissue, or vice versa.

Figure 1C illustrates an embodiment in which a first location 110 spaced-apart from the reference location 10 is within the tissue of the melanoma M. One or more in vivo lactate and/or pyruvate sensing elements are positioned at the reference and first locations 10 and 110, and lactate and/or pyruvate signals are obtained and assessed, and a determination of the type of tissue is made. For example, where a lactate and/or pyruvate signal obtained at the first location 110 is not different (that does not differ by a predetermined amount, e.g., 5%) from a lactate and/or pyruvate signal obtained at the reference location 10, the tissue at the first location 110 is determined to be part of the melanoma M. The magnitude of difference between the signals obtained at the reference and first locations 10 and 110 determines if the tissue at the first location 110 is cancerous tissue of the melanoma M or is outside the melanoma M. This determination can be based on predetermined data or data obtained in real time from other lactate and/or pyruvate sensing elements positioned in or near the melanoma M. For example, the magnitude of difference between the signals may be compared to a reference value which reference value is the difference in lactate and/or pyruvate signals between known cancerous and non-cancerous tissue. If the magnitude of difference between the signals, is similar to or higher than the reference value then the first location 110 is determined to be located outside the melanoma M. If the magnitude of difference between the signals is lower than the reference value, then first location 110 is determined to be located within the melanoma M. In the embodiment depicted in Figure 1C, the first location 110 is located in the melanoma M and the lactate and/or pyruvate signals are not substantially different between the reference and first locations 10 and 110. A second location 120 in the direction D1 in which the lactate and/or pyruvate signal is not substantially changing is selected and a lactate and/or pyruvate signal is detected at the second location 120. The region between the first and second locations 110 and 120 is determined a melanoma boundary 130 if the lactate and/or pyruvate signal obtained at the second location 120 is lower compared to the lactate and/or pyruvate signal obtained at the first location 110.

In Figures 1B and 1C, the locations may be at a predetermined depth relative to the skin surface, which depths are the same. In addition, a third direction into the tissue may be selected to assess the depth in the skin to which the tissue of the melanoma M extends.

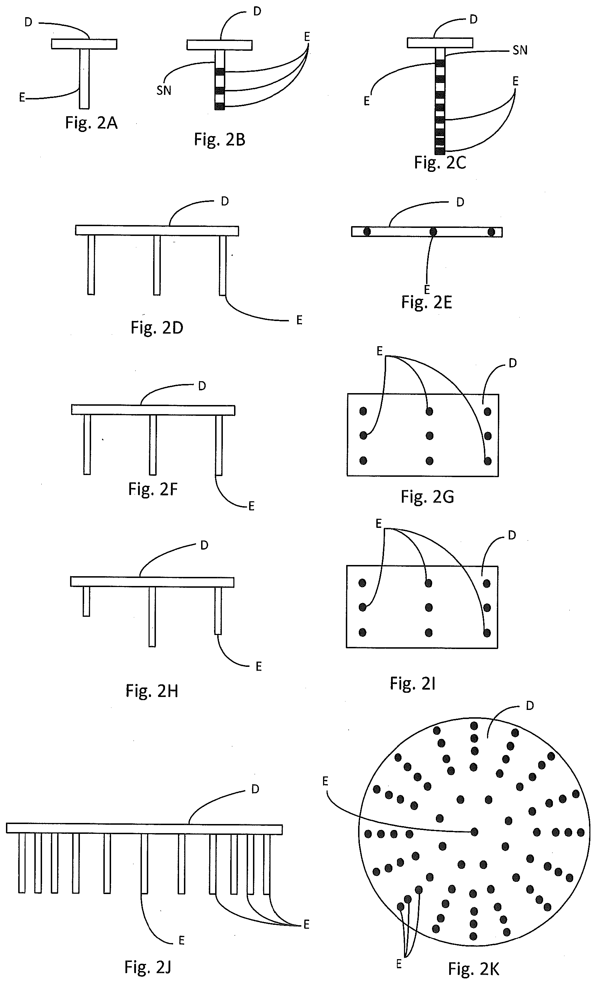

In certain embodiments, as shown in Figures 2A through 2K, a plurality of in vivo lactate and/or pyruvate signals may be measured using one or more lactate and/or pyruvate sensing devices. An in vivo lactate and/or pyruvate sensing device D may have a single lactate and/or pyruvate sensing element E (Figures 2A) that may be moved to the different in vivo locations. In other embodiments, the plurality of lactate and/or pyruvate signals may be measured using an in vivo lactate and/or pyruvate sensing device D that includes a plurality of lactate and/or pyruvate sensing elements E (Figures 2B through 2K), which also may be moved to different locations after each sensing . In either case, the device D may be planar or non-planar. For example, a device D may include a lactate and/or pyruvate sensing needle SN that includes one or a plurality of lactate and/or pyruvate sensing elements E located along the length of the needle SN (Figure 2B and 2C). In another embodiment, the device D may include a plurality of lactate and/or pyruvate sensing elements E arranged in a one-dimensional (Figures 2D and 2E), a two-dimensional (Figures 2F and 2G) or a three-dimensional array (Figures 2H and 2I; which include sensing elements E of differing lengths). For example, the device D may include a grid of a plurality of sensing elements. Such one-, two- or three- dimensional grids of lactate and/or pyruvate sensing elements E may be used to partially or completely blanket or cover tissue such as tissue at least suspected as being cancerous tissue and an area adjacent to the at least suspected cancerous tissue, detect lactate and/or pyruvate signals at these locations and thereby map a perimeter of the cancerous tissue. The number of lactate and/or pyruvate sensing elements E used for assessing tissue may depend on the size of the tumor. In certain embodiments, the number of lactate and/or pyruvate sensing elements present in a device may range from 2-100, e.g., 2-96, 4-84, 8-72, 12-64, 24-54, such as, 4, 8, 16, 32, or 64. In certain embodiments, the placement of the lactate and/or pyruvate sensing elements in a device for assessing tissue in vivo may be uniform. In other cases, the lactate and/or pyruvate sensing elements may be non-uniform, e.g., positioned closer together at a distal portion of the device. For example, the distal portion of a needle shaped device may be the end that is inserted deeper into a tissue compared to a proximal end which is present closer to the surface of the tissue (Figure 2C). In some cases, the peripheral portion of a grid-shaped device may include more lactate and/or pyruvate sensing elements than a central portion of the device D (Figures 2J and 2K). Closer placement of lactate and/or pyruvate sensing elements may provide lactate and/or pyruvate signals at locations closer to a boundary between cancerous and non-cancerous tissue to fine tune the mapping of the edges of the cancerous tissue.

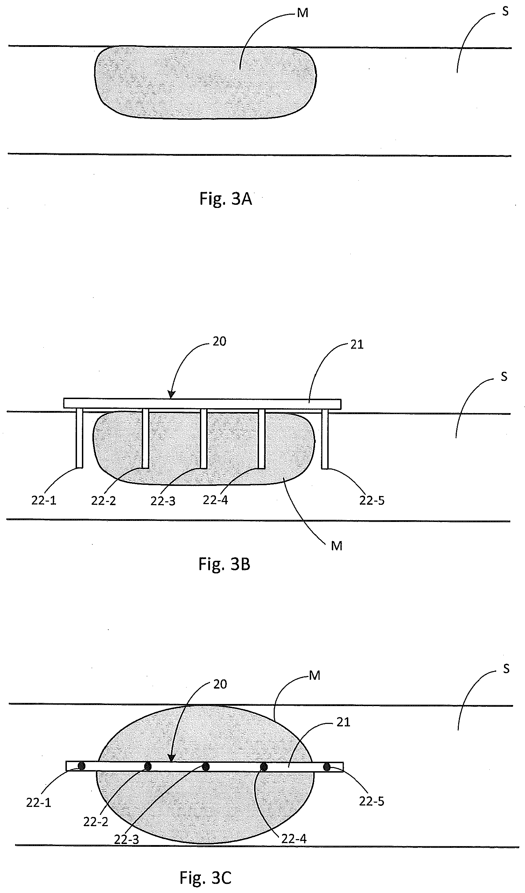

A schematic of an example device that includes a plurality of lactate and/or pyruvate sensing elements is provided in Figures 3B and 3C. In Figures 3A and 3B, a cross section (shown along a depth in the skin tissue) of a melanoma M present in skin tissue S is depicted. Figure 3B shows a lactate and/or pyruvate sensing device 20 inserted into the melanoma M. The in vivo lactate and/or pyruvate sensing device 20 includes a body 21 and a plurality of lactate and/or pyruvate sensing elements 22-1 through 22-5 extending from the body 21 and which are placed at a first depth within the skin, and may not all be in the same dimension. The depths of each sensing element may all be the same as shown here, or one or more may differ. Figure 3C is a view of the device shown in Figure 3B as seen on the surface of the skin S.

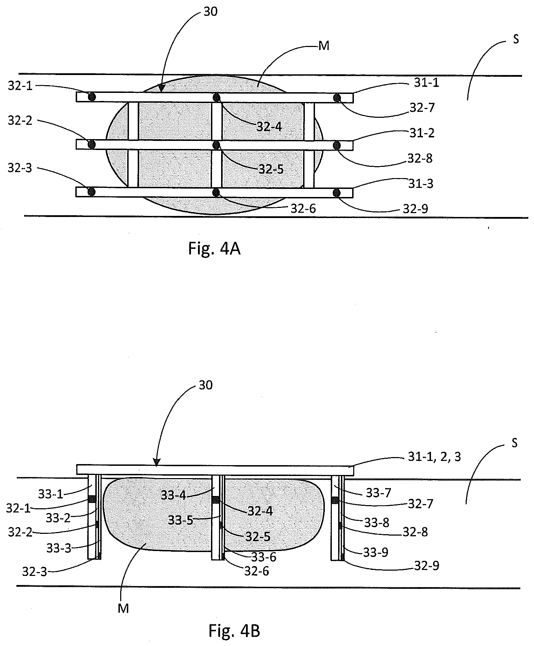

Figures 4A and 4B are schematics of an in vivo lactate and/or pyruvate sensing device 30 containing a plurality of sensing elements 32-1 through 32-9 arranged in a two-dimensional array, e.g., as a uniform grid, and extending from first, second and third body members 31-1, 31-2, and 31-3. In Figure 3A, a surface view of the device 30 placed in the skin S of a subject is depicted. In certain embodiments, each of the sensing elements 32-1 through 32-9 may be placed on insertable members 33-1 through 33-9 of the device 30 which insertable members 33-1 through 33-9 are of the same length and the lactate and/or pyruvate sensing elements 32-1 through 32-9 may all be placed at the same position relative to the length of the insertable members. Thus, the sensing elements 32-1 through 32-9 may detect lactate and/or pyruvate signals at a same depth inside the tissue. In other embodiments, the insertable members 33-1 through 33-9 may include lactate and/or pyruvate sensing elements 32-1 through 32-9 at different locations along their length such that they detect lactate and/or pyruvate signals at different depths in the tissue. An embodiment of such a device is depicted in Figure 3B. In Figure 3B, a side view of a grid shaped device 30 which has lactate and/or pyruvate sensing elements 32-1 through 32-9 is depicted. The position of the lactate and/or pyruvate sensing element 32-1 is positioned on insertable member 33-1 closest to the skin surface and position of the sensing element 32-3 positioned on insertable member 33-3 is farthest from the skin surface. The same pattern is true for lactate and/or pyruvate sensing elements 32-4 through 32-6 and for 32-7 through 32-9.

In certain embodiments, the lactate and/or pyruvate signal at a first location may be used to identify cancerous tissue when the signal is above a threshold level. As noted above, the threshold signal may be a signal that distinguishes cancerous tissue from non-cancerous tissue, such that a lactate and/or pyruvate signal above the threshold signal indicates cancerous tissue and below a threshold signal indicates non-cancerous or normal tissue.

In certain embodiments, instead of or in addition to ascertaining the direction of locations in a tissue along which there is a differential lactate and/or pyruvate signal-increase or decrease, the in vivo method of assessing tissue in a subject may involve comparing the detected signals to the threshold level and identifying the location associated with the signal as cancerous or non-cancerous based on the comparison.

For example, an embodiment of an in vivo method of assessing tissue in a subject may involve sensing lactate and/or pyruvate signals at a plurality of in vivo locations in the tissue using one or more in vivo lactate and/or pyruvate sensing elements, obtaining from the one or more sensing elements data comprising sensing element location and the lactate and/or pyruvate signals, correlating the lactate and/or pyruvate signals to cancerous tissue or non-cancerous tissue, and identifying cancerous and non-cancerous tissue locations based on sensing element location and the correlated lactate and/or pyruvate signals, and analyzing the identified tissue to determine a boundary of cancerous tissue relative to non-cancerous tissue. Embodiments may include generating a map of the sensed area and displaying it on a display device.

In certain cases, the first in vivo lactate and/or pyruvate sensing element may identify the presence and/or concentration of cancerous tissue at a first insertion location and the method may further include positioning a second in vivo lactate and/or pyruvate sensing element in a second insertion location spaced apart from the first insertion location, wherein the positioning is at an insertion site at a greater distance from a reference point; and obtaining from the second sensing element data that includes sensing element location and lactate and/or pyruvate signal, and correlating the lactate and/or pyruvate signal to cancerous tissue or non-cancerous tissue. The reference point may be a point at a location in the tissue which is known or at least suspected to be cancerous.

In certain cases, the method may further include repeating the positioning, obtaining and correlating until an insertion location is identified having lactate and/or pyruvate signal (or a ratio thereof) that is the same as that of a negative control or is at or lower than the threshold level, indicating the presence of non-cancerous tissue at the particular insertion location. As noted above, the threshold level distinguishes cancerous from non-cancerous tissue. A negative control is tissue known to be noncancerous.

In certain embodiments, the cancerous tissue may have a high lactate and/or pyruvate signal in the central part of the cancerous tissue and may have a relatively low lactate and/or pyruvate signal towards the peripheral part of the cancerous tissue. In certain embodiments, the locations may be identified as having cancerous tissue as the signals, though decreasing, are above the threshold.

In certain embodiments, analyzing the identified tissue to determine a boundary of cancerous tissue relative to non-cancerous tissue may include obtaining the lactate and/or pyruvate signals at the sensor locations to construct a pattern corresponding to the identified tissue. The pattern may be a two-dimensional or a three-dimensional map. The pattern may be displayed on a display device, e.g., for example as a topographical map of the sensed area.

In certain embodiments, the lactate and/or pyruvate sensing element may provide data regarding lactate and/or pyruvate signal and location of the lactate and/or pyruvate sensing element in the tissue of a subject. For example, the lactate and/or pyruvate sensing element may include material that facilitates imaging of the lactate and/or pyruvate sensing element inserted into a tissue to indicate location of the sensing element. In certain embodiments, the data regarding location of the sensing element may be obtained by markings on the sensing element. For example, a device may include lactate and/or pyruvate sensing elements located at certain distances apart on the device, which distances are mapped. The location of the sensing elements in the tissue when the device is inserted into the tissue may be determined by the depth to which the device is inserted and correlating that to the known positions of the sensing elements on the device.

In certain embodiments, a virtual map of the assessed tissue may be created using the locations of the sensing elements and the measured lactate and/or pyruvate signals. The map may depict the edges of the tumor. The map may be generated at certain periods of time during which therapy is being administered to the subject and may be used to assess whether a cancer therapy is effectively reducing the size of the tumor.

In certain embodiments, the map may be used to facilitate biopsy, removal of the tumor and localized treatment of the tumor. In certain embodiments, the perimeter may be fine-tuned to increase or decrease the perimeter or clarify the contours of a tumor. For example, in instances where it is desirable to remove the tumor but not any unintended non-cancerous cells adjacent to the tumor, additional lactate and/or pyruvate signal measurements may be made to accurately determine a decrease in the perimeter within which the cancer cells are located.

In certain embodiments, the methods, devices and systems herein may be used in conjunction with a surgical procedure to remove cancerous tissue from a tissue/organ. For example, a scalpel may be used to make an incision in a first location identified as having cancer cells and then used to extend the incision to a second location identified as having cancer cells and so on in order to excise the tumor tissue out of the tissue.

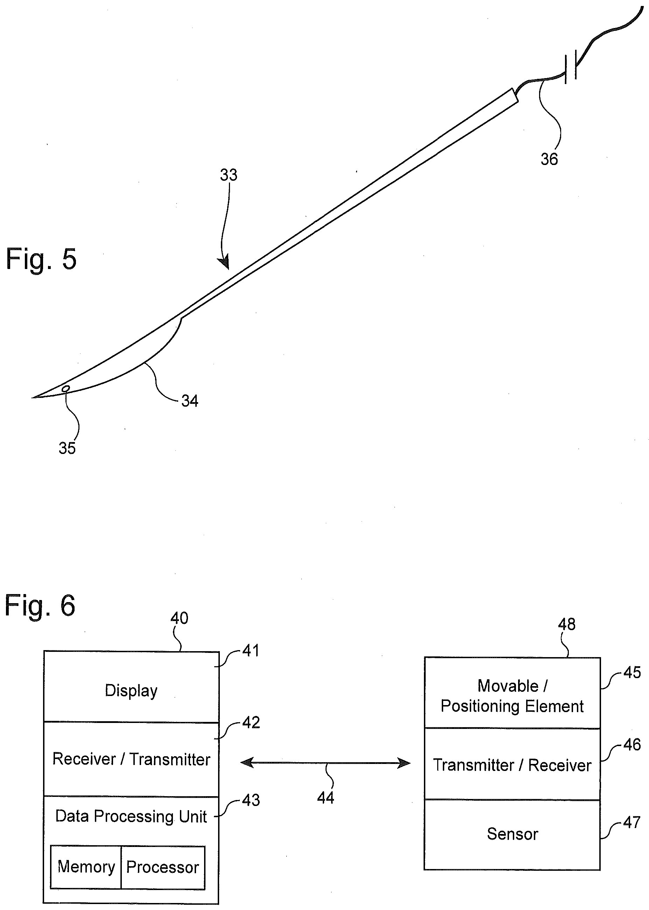

In another embodiment, a scalpel (33) may include one or more lactate and/or pyruvate sensing elements (35) at, for example, the leading edge of the blade (34) that may provide data regarding position of the scalpel in a tissue and lactate and/or pyruvate signal at that position as exemplified in Fig. 5. The data may be communicated to a processing device wirelessly or via a wired communication element (36), such as exemplified in Fig. 5. The scalpel may be positioned adjacent a cancerous tissue; the scalpel may be moved along a perimeter of the cancerous tissue while simultaneously detecting a level of lactate and/or pyruvate to form an incision to separate cancerous tissue from non-cancerous tissue. The scalpel may be moved along the perimeter of the cancerous tissue until the cancerous tissue is completely separated from the non-cancerous tissue.

In some cases, the scalpel may be used to make a small incision at a first location in the tissue, determine lactate and/or pyruvate signal at the first location, extend the incision in a first direction when a lactate and/or pyruvate signal indicative of cancerous tissue is detected, stop the extension of the incision when a lactate and/or pyruvate signal indicative of non-cancerous tissue is detected. The scalpel may be repositioned at the first location and used to extend the incision in a direction different from the first direction and the lactate and/or pyruvate signal monitored. The scalpel may be stopped when a lactate and/or pyruvate signal indicative of non-cancerous tissue is detected.

In certain cases, the scalpel may be operatively connected to a device that monitors the lactate and/or pyruvate signal and position of the scalpel in the tissue. The connection may be wireless or wired. In certain embodiments, the scalpel device may provide feedback when it determines a certain type of tissue is contacted by the scalpel, such as cancerous or non cancerous tissue. The device may provide feedback such as an audible or other (e.g., tactile, such as vibratory) alert to provide indication that the incision has reached non-cancerous tissue (or cancerous as the case may be). In certain embodiments, the scalpel device may be a touch sensitive device. For example, the scalpel may be a haptic feedback device, e.g., using an electroactive polymer. The scalpel may include a circuit to return signals conveying haptic information from a force sensor and lactate and/or pyruvate (cancerous/non cancerous) tissue location sensor integrated into the scalpel. The feedback may notify a user, such as, a surgeon operating the scalpel device, to stop and reposition the scalpel, if required. The scalpel device may be operatively connected to a robotic arm that is guiding the scalpel and may be configured to stop the robotic arm when an incision is extending into non-cancerous tissue. The device may reposition the scalpel, if needed.

In certain embodiments, a map of the cancerous region in a tissue may be used to locally deliver therapy to the tumorous region which therapy may be toxic to non-cancerous cells in the tissue. Such a therapy may include radiation, such as, ionizing radiation, chemotherapy and the like.

A first location at which a lactate and/or pyruvate sensing element may be inserted to measure a lactate and/or pyruvate signal may be a region of tissue likely to include cancerous tissue. In other embodiments, a first location at which a lactate and/or pyruvate sensing element may be inserted to measure a lactate and/or pyruvate signal may be a region of tissue likely to include non-cancerous tissue. A location of cancerous tissue may be determined by any standard method known in the art. For example, the method may include determining the location of the cancer tissue by palpation, biopsy, Raman spectroscopy, imaging techniques such as, X-Ray, CT scan, nuclear imaging, ultrasound, magnetic resonance imaging, digital mammography, colonoscopy, virtual colonoscopy, and the like.

As used herein, cancerous tissue refers to tumor such as malignant tissue. Cancerous tissue includes tumors such as metastatic tumors. Any type of cancerous tissue may be assessed using the methods, devices and systems disclosed herein. Example cancers include sarcomas, carcinomas, and lymphomas, melanomas, for example, tumor of the skin, brain, breast, kidney, lungs, gastrointestinal tract: e.g., stomach or colon, lymph node, etc.

As used herein, a subject in which a tissue is assessed by the disclosed methods, devices, and/or systems, may be a mammal, such as, a dog, a cat, a horse, or a human who has been diagnosed as having cancer.

Example lactate and/or pyruvate sensing elements may include two or more electrodes for detecting a lactate and/or pyruvate signal. In certain embodiments, the lactate and/or pyruvate sensing element may include at least a working electrode and a counter electrode. The lactate and/or pyruvate sensing element may include a lactate- or pyruvate responsive enzyme (e.g., lactate and/or pyruvate oxidase or lactate dehydrogenase). In certain embodiments, the lactate-responsive enzyme may be disposed on the working electrode. The lactate and/or pyruvate sensing element may include a redox mediator disposed in proximity to the enzyme, for example, on the working electrode. In certain embodiments, the enzyme may be immobilized on the working electrode via a polymer. In certain embodiments, the redox mediator may be immobilized on the working electrode via a polymer. The sensing element may include a flux-limiting membrane. For example, the flux-limiting membrane may be disposed on a surface of the working electrode. The mediator may be organometallic redox species such as metallocenes including ferrocene or inorganic redox species such as hexacyanoferrate (III), ruthenium hexamine, etc. Additional suitable electron transfer agents usable as redox mediators include osmium transition metal complexes with one or more ligands, each ligand having a nitrogen-containing heterocycle such as 2,2'-bipyridine, 1,10-phenanthroline, 1-methyl, 2-pyridyl biimidazole, or derivatives thereof. The electron transfer agents may also have one or more ligands covalently bound in a polymer, each ligand having at least one nitrogen-containing heterocycle, such as pyridine, imidazole, or derivatives thereof.

In example embodiments, the lactate and/or pyruvate sensing element detects and/or measures lactate and/or pyruvate signal immediately following positioning of the element in a tissue. The lactate and/or pyruvate sensing elements may require little or no equilibration time after in vivo placement, and require no user intervention for calibration, i.e., it may be a no user calibration sensor -also referred to as factory-only calibrated sensor. For example, the sensing element may detect a lactate and/or pyruvate signal within 5 min of placement in a tissue, e.g., 4 min, 3 min, 2 min, 1 min, 30 sec, 15 sec, 10 sec, 5 sec, 1 sec, or less. The sensing and processing can therefore be accomplished in real time, e.g., while a subject is undergoing surgery, so that the surgical time isn't increased because of the lactate and/or pyruvate sensing and analysis.

In some embodiments, the device containing lactate and/or pyruvate sensing elements may be positioned transcutaneously. In other embodiments, the device containing lactate and/or pyruvate sensing elements may be wholly implanted inside a subject. The device may be transcutaneously positioned or wholly implanted for a short period of time during which cancerous tissue is assessed using the methods and/or devices and/or systems disclosed herein. In other cases, the in vivo sensing device may be transcutaneously positioned or wholly implanted for a longer period of time, for example, several days prior to a surgical procedure for treatment of the cancerous tissue. For example, the in vivo sensing device may be transcutaneously positioned or wholly implanted in a tissue about 1 month, 2 weeks, 7 days, 3 days, 1 day, 16 hours, 8 hours, 4 hours, 1 hour prior to a surgery or application of treatment to treat cancerous tissue (removal, radiation, chemotherapy, etc.), or prior to assessing of the tissue to determine a boundary between cancerous and non-cancerous tissue.

The lactate and/or pyruvate sensing element may include a substrate, e.g., a planar substrate, on which the working and counter electrodes are disposed. In certain embodiments, the lactate and/or pyruvate sensing element is a wire, e.g., a working electrode wire inner portion with one or more other electrodes associated (e.g., on, including wrapped or twisted around) therewith. The counter electrode may work as a counter/reference electrode. Example lactate and/or pyruvate sensing elements are known in the art and may include those described in US7,462,264; US6,284,478; and WO2009135197.

The lactate and/or pyruvate signal and lactate and/or pyruvate sensing elements used therefor may be electrochemical or optical in nature. Embodiments include electrochemical lactate and/or pyruvate sensors and optical lactate and/or pyruvate sensors. The lactate and/or pyruvate signal may be selected from the group consisting of current, impedance, voltage, resistance, capacitance, or combination thereof. In some embodiments, a fiber optic lactate and/or pyruvate sensor is used. In certain embodiments, the lactate and/or pyruvate sensing elements may be continuous lactate and/or pyruvate sensors that detect lactate and/or pyruvate signals periodically over a period of time automatically. A continuous lactate and/or pyruvate sensor may detect a lactate and/or pyruvate signal every 1 second, 3 seconds, 10 seconds, 20 seconds, 30 seconds, 45 seconds, 1 min, 5 min, 10 min, and so on. Signal obtained may then be transmitted to a processor for processing- either wirelessly (RF, Bluetooth, or the like) or with a wire.

The lactate and/or pyruvate sensing element may convert lactic and/or pyruvic acid using the lactate and/or pyruvate responsive enzyme and measure a signal associated with the conversion. A value for the signal associated with the working electrode may be determined. If multiple working electrodes are used, signal values from each of the working electrodes may be determined. A microprocessor may be used to collect these signal values and/or to further process these values. In certain embodiments, the lactate and/or pyruvate signal may be used to determine a concentration of lactate and/or pyruvate at the insertion location of the lactate and/or pyruvate sensing element.

In certain embodiments, the device for assessing a tissue located in body of a subject may include one or more lactate and/or pyruvate sensing elements and may be operatively connected to a system that includes a processor and that determines location of the lactate and/or pyruvate sensing elements in the tissue, compares the lactate and/or pyruvate signals either to each other or to the reference signal measured at a cancerous portion of the tissue, determines the spatial direction in which the lactate and/or pyruvate signals are increasing or decreasing, and identifies the boundary of the cancerous tissue.

The devices used in the in vivo methods disclosed herein may be part of a system that may be automated or semi-automated. In certain embodiments, the system used to perform the present in vivo methods may include lactate and/or pyruvate sensing elements, a memory, a processor, and optionally a display. In certain embodiments, the system may transmit (e.g., wirelessly or with a wire) a result of the determining a boundary between cancerous or non-cancerous tissue in a subject to a display. In certain embodiments, the system for determining a boundary between cancerous and non-cancerous tissue may include a processor operatively connected to a memory and to a positioning element for determining locations of two or more lactate and/or pyruvate sensing elements positioned in vivo in a tissue of a subject, the memory may include instructions stored therein which when executed by the processor cause the processor to perform the methods disclosed herein. For example the processor may determine the locations of the two or more lactate and/or pyruvate sensing elements using the positioning system, determine a difference lactate and/or pyruvate signal from the two or more lactate and/or pyruvate sensing elements, determine a direction of increase or decrease in lactate and/or pyruvate signals between the locations at which the lactate and/or pyruvate sensing elements are located, and output the direction of increase or decrease in lactate and/or pyruvate signals. The processor may be programmed to determine a boundary between cancerous tissue and non-cancerous tissue by correlating the direction of increase or decrease to the position of the locations. The processor may be programmed to display a map depicting a boundary between cancerous tissue and non-cancerous tissue. The map may be a two-dimensional or a three-dimensional map depicting cancerous tissue and non-cancerous tissue.

An example of such a system is shown in Fig. 6. The exemplary system includes a processing component (40) including a data processing unit (43) having a processor and memory, operatively coupled to display (41) and a receiver/transmitter unit (42) that is in communication (44) with a receiver/transmitter unit (46) of a sensing component (48). The sensing component further includes a movable positioning element (45) for automated movement, and a sensor (47). The communication (44) between the processing component (40) and the sensing component (48) may be wired or wireless.

The positioning element/system may be configured to track the locations of the lactate and/or pyruvate sensing elements in a tissue in a subject. The positioning system may include an imaging system, an electromagnetic system, or a combination thereof for tracking the location of insertion of a lactate and/or pyruvate sensing element in a tissue in a subject. Example imaging systems include, camera, computed tomography (CT), magnetic resonance imaging (MRI), radiography, X-Ray, isocentric C-arm fluoroscopic imaging, positron emission tomography (PET), and ultrasound imaging and the like. In certain cases, the location of a lactate and/or pyruvate sensing element may be determined by detecting an electromagnetic field associated with the lactate and/or pyruvate sensing elements. For example, the lactate and/or pyruvate sensing elements may include conductive elements, such as, electrodes that produce an electromagnetic field around the lactate and/or pyruvate sensing element which field may be detected and used to determine location of the lactate and/or pyruvate sensing element in a tissue in vivo.

In certain embodiments, the system may include a controller operatively coupled to the processor, wherein the controller controls movement of the movable positioning element of the lactate and/or pyruvate sensing elements. In certain cases, the processor may cause the movable positing element to change the location of one or more lactate and/or pyruvate sensing elements, which location may be determined by the processor based on information obtained from the positioning system.

In certain embodiments, a lactate and/or pyruvate sensing element of the present disclosure may provide a lactate and/or pyruvate signal to the processor which may store the signal in the memory. A lactate and/or pyruvate signal may be transmitted to the processor and/or the memory via a wired or wireless connection. Wireless protocols that can be used include Wi-Fi, near field communication (NFC), radio frequency identification (RFID), Bluetooth, or Bluetooth Low Energy, to name a few.

The various illustrative processes described in connection with the embodiments herein may be implemented or performed with a general purpose processor, a Digital Signal Processor (DSP), an Application Specific Integrated Circuit (ASIC), a Field Programmable Gate Array (FPGA) or other programmable logic device, discrete gate or transistor logic, discrete hardware components, or any combination thereof designed to perform the functions described herein. A general purpose processor may be a microprocessor, but in the alternative, the processor may be any conventional processor, controller, microcontroller, or state machine. The processor can be part of a computing system that also has a user interface port that communicates with a user interface, and which receives commands entered by a user, has at least one memory (e.g., hard drive or other comparable storage, and random access memory) that stores electronic information including a program that operates under control of the processor and with communication via the user interface port, and a video output that produces its output via any kind of video output format, e.g., VGA, DVI, HDMI, DisplayPort, or any other form.

A processor may also be implemented as a combination of computing devices, e.g., a combination of a DSP and a microprocessor, a plurality of microprocessors, one or more microprocessors in conjunction with a DSP core, or any other such configuration. These devices may also be used to select values for devices as described herein. The camera may be a digital camera of any type including those using CMOS, CCD or other digital image capture technology.

The steps of a method or algorithm described in connection with the embodiments disclosed herein may be embodied directly in hardware, in a software module executed by a processor, or in a combination of the two. A software module may reside in Random Access Memory (RAM), flash memory, Read Only Memory (ROM), Electrically Programmable ROM (EPROM), Electrically Erasable Programmable ROM (EEPROM), registers, hard disk, a removable disk, a CD-ROM, or any other form of storage medium known in the art. An exemplary storage medium is coupled to the processor such that the processor can read information from, and write information to, the storage medium. In the alternative, the storage medium may be integral to the processor. The processor and the storage medium may reside in an ASIC. The ASIC may reside in a user terminal. In the alternative, the processor and the storage medium may reside as discrete components in a user terminal.

In one or more example embodiments, the functions described may be implemented in hardware, software, firmware, or any combination thereof. If implemented in software, the functions may be stored on, transmitted over or resulting analysis/calculation data output as one or more instructions, code or other information on a computer-readable medium. Computer-readable media includes both computer storage media and communication media including any medium that facilitates transfer of a computer program from one place to another. A storage media may be any available non-transitory media that can be accessed by a computer. By way of example, such computer-readable media can include RAM, ROM, EEPROM, CD-ROM or other optical disk storage, magnetic disk storage or other magnetic storage devices, or any other medium that can be used to carry or store desired program code in the form of instructions or data structures and that can be accessed by a computer. The memory storage can also be rotating magnetic hard disk drives, optical disk drives, or flash memory based storage drives or other such solid state, magnetic, or optical storage devices. Disk and disc, as used herein, includes compact disc (CD), laser disc, optical disc, digital versatile disc (DVD), floppy disk and Blu-ray disc where disks usually reproduce data magnetically, while discs reproduce data optically with lasers. Combinations of the above should also be included within the scope of computer-readable media.

To the extent the embodiments disclosed herein include or operate in association with memory, storage, and/or computer readable media, then that memory, storage, and/or computer readable media are non-transitory. Accordingly, to the extent that memory, storage, and/or computer readable media are covered by one or more claims, then that memory, storage, and/or computer readable media is only non-transitory.

As noted herein, the devices herein may be part of a surgical instrument that excises the tumor. In certain embodiments, the device may include an injection device for injecting a therapeutic agent. The device may be configured for injecting the therapeutic agent at locations having a lactate and/or pyruvate signal higher than the threshold lactate and/or pyruvate signal. Any therapeutic agent known to be efficacious against the cancer present in the tissue may be used. The therapeutic agent may be a chemotherapeutic agent, a cancer antigen specific antibody, or a combination thereof.

All features, elements, components, functions, and steps described with respect to any embodiment provided herein are intended to be freely combinable and substitutable with those from any other embodiment. If a certain feature, element, component, function, or step is described with respect to only one embodiment, then it should be understood that that feature, element, component, function, or step can be used with every other embodiment described herein unless explicitly stated otherwise. This paragraph therefore serves as antecedent basis and written support for the introduction of claims, at any time, that combine features, elements, components, functions, and steps from different embodiments, or that substitute features, elements, components, functions, and steps from one embodiment with those of another, even if the following description does not explicitly state, in a particular instance, that such combinations or substitutions are possible. Express recitation of every possible combination and substitution is overly burdensome, especially given that the permissibility of each and every such combination and substitution will be readily recognized by those of ordinary skill in the art upon reading this description.

While the embodiments are susceptible to various modifications and alternative forms, specific examples thereof have been shown in the drawings and are herein described in detail. It should be understood, however, that these embodiments are not to be limited to the particular form disclosed, but to the contrary, these embodiments are to cover all modifications and alternatives falling within the disclosure.

## Claims

1. A system for monitoring a boundary between cancerous and non-cancerous tissue, the system comprising a processor operatively connected to a non-transitory memory, two or more sensing elements (22-1122-5, 32-1132-9)
in communication with the processor and configured to be positioned in vivo in a tissue of the skin of a subject, wherein each of the two or more sensing elements is configured to sense lactate to provide lactate signals or each of the two or more sensing elements is configured to sense pyruvate to provide pyruvate signals, and a positioning element (45) configured to determine locations of the two or more sensing elements;
the memory comprising instructions which are executed by the processor to:
determine, using the positioning element, the positions of the locations of the two or more sensing elements;
determine a difference between the signals obtained from each of the two or more sensing elements;
determine a direction of increase or decrease in the signals between the locations at which the sensing elements are located; determine a boundary between cancerous tissue and non-cancerous tissue by correlating the direction of increase or decrease to the position of the locations; and
output the direction of increase or decrease in the signals.

2. The system of claim 1, wherein the two or more sensing elements are electrochemical.

3. The system of any preceding claim, wherein the processor is programmed to display a map depicting the boundary between cancerous tissue and non-cancerous tissue.

4. The system of any preceding claim, wherein the processor is programmed to display two-dimensional map depicting cancerous tissue and non-cancerous tissue.

5. The system of any preceding claim, wherein the processor is programmed to display three-dimensional map depicting cancerous tissue and non-cancerous tissue.

6. The system of any preceding claim, wherein the positioning system is configured to track the locations of the sensing elements.

7. The system of any preceding claim, wherein the positioning system is configured to track the locations of the sensing elements and a surgical instrument.

8. The system of any preceding claim, wherein the positioning system is configured to track the locations of the sensing elements, wherein the sensing elements are located on one or more surgical devices.

9. The system of claim 8, wherein the positioning system is configured to track the one or more surgical devices.

10. The system of any preceding claim, wherein the positioning system comprises an imaging system.

11. The system of any preceding claim, wherein the positioning system comprises an electromagnetic tracking system.

12. The system of any preceding claim, wherein the system further comprises a controller operatively coupled to the processor, wherein the controller controls movement of the sensing elements.

## Patentansprüche

1. System zum Überwachen einer Grenze zwischen kanzerösem und nicht-kanzerösem Gewebe, wobei das System einen Prozessor, der operativ mit einem nicht-flüchtigen Speicher verbunden ist, zwei oder mehr Erfassungselemente, die mit dem Prozessor in Verbindung stehen und konfiguriert sind, um in vivo in einem Gewebe der Haut eines Subjekts positioniert zu werden, wobei jedes der zwei oder mehr Erfassungselemente konfiguriert ist, um Laktat zu erfassen, um Laktatsignale bereitzustellen, oder jedes der zwei oder mehr Erfassungselemente konfiguriert ist, um Pyruvat zu erfassen, um Pyruvatsignale bereitzustellen, und ein Positionierungselement (45), das konfiguriert ist, um Positionen der zwei oder mehr Erfassungselemente zu bestimmen, aufweist;
wobei der Speicher Anweisungen aufweist, die durch den Prozessor ausgeführt werden, um:
unter Verwendung des Positionierungselements die Positionen der Orte der zwei oder mehr Erfassungselemente zu bestimmen;
eine Differenz zwischen den Signalen, die von jedem der zwei oder mehr Erfassungselemente erhalten werden, zu bestimmen;
eine Grenze zwischen kanzerösem Gewebe und nicht-kanzerösem Gewebe durch Korrelieren der Richtung der Zunahme oder Abnahme mit der Position der Orte zu bestimmen; und
die Richtung der Zunahme oder Abnahme in den Signalen auszugeben.

2. System nach Anspruch 1, wobei die zwei oder mehr Erfassungselemente elektrochemisch sind.

3. System nach einem der vorhergehenden Ansprüche, wobei der Prozessor programmiert ist, um eine Karte anzuzeigen, die die Grenze zwischen kanzerösem Gewebe und nicht-kanzerösem Gewebe darstellt.

4. System nach einem der vorhergehenden Ansprüche, wobei der Prozessor programmiert ist, um eine zweidimensionale Karte anzuzeigen, die kanzeröses Gewebe und nicht-kanzeröses Gewebe darstellt.

5. System nach einem der vorhergehenden Ansprüche, wobei der Prozessor programmiert ist, um eine dreidimensionale Karte anzuzeigen, die kanzeröses Gewebe und nicht-kanzeröses Gewebe darstellt.

6. System nach einem der vorhergehenden Ansprüche, wobei das Positionierungssystem konfiguriert ist, um die Orte der Erfassungselemente zu verfolgen.

7. System nach einem der vorhergehenden Ansprüche, wobei das Positionierungssystem konfiguriert ist, um die Orte der Erfassungselemente und eines chirurgischen Instruments zu verfolgen.

8. System nach einem der vorhergehenden Ansprüche, wobei das Positionierungssystem konfiguriert ist, um die Orte der Erfassungselemente zu verfolgen, wobei die Erfassungselemente auf einer oder mehreren chirurgischen Vorrichtungen angeordnet sind.

9. System nach Anspruch 8, wobei das Positionierungssystem konfiguriert ist, um die eine oder mehreren chirurgischen Vorrichtungen zu verfolgen.

10. System nach einem der vorhergehenden Ansprüche, wobei das Positionierungssystem ein Bildgebungssystem aufweist.

11. System nach einem der vorhergehenden Ansprüche, wobei das Positionierungssystem ein elektromagnetisches Verfolgungssystem aufweist.

12. System nach einem der vorhergehenden Ansprüche, wobei das System ferner eine Steuerung aufweist, die operativ mit dem Prozessor gekoppelt ist, wobei die Steuerung die Bewegung der Erfassungselemente steuert.

## Revendications

1. - Système destiné à surveiller une frontière entre un tissu cancéreux et un tissu non cancéreux, le système comprenant un processeur relié de manière fonctionnelle à une mémoire non transitoire, au moins deux éléments de détection (22-1/22-5, 32-1/32-9) en communication avec le processeur et configurés pour être positionnés in vivo dans un tissu de la peau d'un sujet, chacun des au moins deux éléments de détection étant configuré pour détecter le lactate afin de fournir des signaux de lactate, ou chacun des au moins deux éléments de détection étant configuré pour détecter le pyruvate afin de fournir des signaux de pyruvate, et un élément de localisation (45) configuré pour déterminer les emplacements des au moins deux éléments de détection ;
la mémoire comprenant des instructions qui sont exécutées par le processeur pour :
déterminer, à l'aide de l'élément de localisation, les positions des emplacements des au moins deux éléments de détection ;
déterminer une différence entre les signaux obtenus à partir de chacun des au moins deux éléments de détection ;
déterminer un sens d'augmentation ou de diminution dans les signaux entre les emplacements auxquels se trouvent les éléments de détection ;
déterminer une frontière entre un tissu cancéreux et un tissu non cancéreux en établissant une corrélation entre le sens d'augmentation ou de diminution et la position des emplacements ; et
délivrer le sens d'augmentation ou de diminution dans les signaux.

2. - Système selon la revendication 1, dans lequel les au moins deux éléments de détection sont électrochimiques.

3. - Système selon l'une quelconque des revendications précédentes, dans lequel le processeur est programmé pour afficher une carte représentant la frontière entre le tissu cancéreux et le tissu non cancéreux.

4. - Système selon l'une quelconque des revendications précédentes, dans lequel le processeur est programmé pour afficher une carte bidimensionnelle représentant le tissu cancéreux et le tissu non cancéreux.

5. - Système selon l'une quelconque des revendications précédentes, dans lequel le processeur est programmé pour afficher une carte tridimensionnelle représentant le tissu cancéreux et le tissu non cancéreux.

6. - Système selon l'une quelconque des revendications précédentes, dans lequel le système de localisation est configuré pour suivre les emplacements des éléments de détection.

7. - Système selon l'une quelconque des revendications précédentes, dans lequel le système de localisation est configuré pour suivre les emplacements des éléments de détection et un instrument chirurgical.

8. - Système selon l'une quelconque des revendications précédentes, dans lequel le système de localisation est configuré pour suivre les emplacements des éléments de détection, les éléments de détection étant situés sur un ou plusieurs dispositifs chirurgicaux.

9. - Système selon la revendication 8, dans lequel le système de localisation est configuré pour suivre le ou les dispositifs chirurgicaux.

10. - Système selon l'une quelconque des revendications précédentes, dans lequel le système de localisation comprend un système d'imagerie.

11. - Système selon l'une quelconque des revendications précédentes, dans lequel le système de localisation comprend un système de suivi électromagnétique.

12. - Système selon l'une quelconque des revendications précédentes, dans lequel le système comprend en outre un dispositif de commande couplé de manière fonctionnelle au processeur, le dispositif de commande commandant le mouvement des éléments de détection.
